# EUROPÄISCHE PATENTSCHRIFT

(11) **EP 1 581 613 B1**
(45) Veröffentlichungstag und Bekanntmachung des Hinweises auf die Patenterteilung: **14.04.2010**
(21) Anmeldenummer: 03750533.6
(22) Anmeldetag: 11.09.2003
(51) Int. Cl.: C12M 3/02, C12M 3/00, C12M 3/04

(54) **VERFAHREN ZUR BEHANDLUNG VON ZELLKULTUREN**
METHOD FOR TREATING CELL CULTURES
PROCEDE POUR LE TRAITEMENT DE CULTURES CELLULAIRES

(30) Priorität: 11.09.2002 DE 10242066
(43) Veröffentlichungstag der Anmeldung: 05.10.2005
(73) Patentinhaber: Ngoli, Dominique, 06333 Hettstedt (DE)
(72) Erfinder: Ngoli, Dominique, 06333 Hettstedt (DE)
(74) Vertreter: Hartz, Nikolai
(86) Internationale Anmeldenummer: PCT/EP2003/010147
(87) Internationale Veröffentlichungsnummer: WO 2004/027017

(56) Entgegenhaltungen:
- EP-A- 0 387 975
- US-A- 5 316 945
- GURARI-ROTMAN DALIA ET AL: "Test of a novel transparent floating carrier by recombinant CHO cells expressing sIL6 receptor protein" CYTOTECHNOLOGY, Bd. 37, Nr. 2, 2001, Seiten 75-81, XP009023598 ISSN: 0920-9069
- BISSON ISABELLE ET AL: "Human urothelial cells grown on collagen adsorbed to surface-modified polymers" UROLOGY, Bd. 60, Nr. 1, Juli 2002 (2002-07), Seiten 176-180, XP001156995 ISSN: 0090-4295

## Beschreibung

Die Erfindung betrifft ein Verfahren zur Untersuchung von Zellkulturen, sowie ein System zur Verwendung in einem solchen Verfahren.

Bei der Entwicklung von therapeutischen oder pharmakologischen Verfahren für Zwecke der Humanmedizin ist es häufig in einer frühen Entwicklungsstufe erforderlich, vergleichende Untersuchungen der Einwirkung eines Wirkstoffs auf eine Zellkultur vorzunehmen. Hierzu müssen Zellkulturen unter kontrollierten Bedingungen hergestellt und mit,einem Wirkstoff behandelt werden. Schlüssige Ergebnisse werden dabei gewöhnlich nur dann erhalten, wenn gesonderte (gleiche oder verschiedene) Zellkulturen vergleichend behandelt werden. Dabei treten schwerwiegende Probleme auf. Häufig werden die Zellkulturen während ihrer Vorbereitung oder während der Untersuchung geschädigt oder unter Stress gesetzt, so dass die Untersuchung zu Ergebnissen führt, die zumindest teilweise eine Folge der Schädigung der Zellen sind. Somit kommt es bei einem Rückschluss vom Untersuchungsergebnis an der geschädigten Zelle auf die in vivo Situation zu folgenreichen Fehlern. Außerdem sind die Schädigungen oder Stresssituationen von Zellkulturprobe zu Zellkulturprobe nicht hinreichend reproduzierbar bzw. mit verborgenen Parametern behaftet. Dies führt zu einem großen Anteil an falsch positiven oder falsch negativen Ergebnissen.

Aus diesem Grund kommt den sich an die Zelluntersuchungen anschließenden in vivo Tierversuchen mit Modelltieren wie Mäusen eine große Bedeutung zu. Es besteht also die paradoxe Situation, dass man von Versuchen mit menschlichen Zellkulturen an sich eine hohe Aussagekraft erwarten sollte, die aber wegen der genannten Störungen nicht realisiert wird. Die Untersuchungstechnik macht die an sich zu erwartende Aussagekraft wieder zunichte. Andererseits sind die Tierversuche mit Modelltieren als in vivo Versuche nicht so sehr mit Experimentierartefakten behaftet, dafür aber haben sie von Haus aus nur eine sehr beschränkte Aussagekraft (ganz abgesehen von dem Problem für vergleichende Untersuchungen genetisch identische Tiergruppen zu züchten). Dies hat dazu geführt, dass große Anstrengungen unternommen werden, insbesondere durch extrem kostspielige Genom- oder Proteomvergleiche, die Übertragbarkeit der Ergebnisse von Modelltieren auf den Menschen sicherzustellen. Eine befriedigende Lösung dieses Problems ist derzeit noch in weiter Ferne. Daher ist bei therapeutischen oder pharmakologischen Neuentwicklungen der eigentlich extrem aufwendige "Flaschenhals" die Durchführung und statistische Auswertung klinischer Versuche an Patientenkollektiven. Dies behindert naturgemäß die Entwicklung neuer Behandlungsmethoden in extremem Maße. Das Risiko pharmakologischer Neuentwicklungen wird dabei in den im wirtschaftlichen Sinne teuersten und ethischen Sinne problematischsten Bereich, nämlich den der klinischen Untersuchungen verschoben. Ähnliche Probleme bestehen auch bei der Entwicklung von diagnostischen Untersuchungen an Zellkulturen.

Es ist somit Aufgabe der Erfindung, ein Verfahren zur Durchführung von Untersuchungen an einer Zellkultur zu schaffen, das die Zellen der Zellkultur nicht stark unter Stress setzt oder anderweitig schädigt, damit aussagekräftige Vergleichsergebnisse erhalten werden können, die vorzugsweise direkt auf einen der Zellkultur entsprechenden Organismus übertragen werden können, möglichst unter Vermeidung oder Einschränkung von zwischengeschalteten Versuchen mit Modelltieren. Ferner soll ein Verfahren zum stressfreien Passagieren oder Splitten von Zellen bereitgestellt werden.

Diese Aufgabe wird erfindungsgemäß gelöst durch ein Verfahren zur Behandlung von Zellkulturen, gekennzeichnet durch die folgenden Stufen:
(a) Bereitstellen eines auf einer Oberfläche adhärente Zellen aufweisenden Primärträgers, wobei die Oberfläche für die Adhärenz und das Wachstum der Zellen geeignet ist;
(b) Kultivieren der Zellen auf dem Träger unter vorbestimmten Bedingungen unter Ausbildung einer Zellschicht auf der Oberfläche des Primärträgers;
(c) Abtrennung eines eine Zellschicht tragenden Trägerabschnitts des in Stufe (b) erhaltenen Trägers;
(d) Positionieren des in Stufe (c) abgetrennten Trägerabschnitts in einer solchen Lagebeziehung zu einer für die Adhärenz und das Wachstum der Zellen geeigneten Oberfläche eines Sekundärträgers, dass die Zellen vom Trägerabschnitt auf den Sekundärträger hinüberwachsen können.

Es ist ein besonderer Vorteil der Erfindung, dass die Zellen der flächig wachsenden Zellkultur einer Behandlung und anschließenden Untersuchung unterzogen werden können, ohne dass sie von der Oberfläche entfernt werden müssen. Hierdurch werden Stresssituationen weitgehend vermieden. Ein der Erfindung zugrundeliegendes allgemeines Prinzip besteht darin, bei der Untersuchung und Behandlung der Zellen einen Übergang von dem zweidimensionalen Zustand in einen dreidimensionalen Zustand oder einen Übergang von einem dreidimensionalen Verteilungszustand in einen zweidimensionalen Verteilungszustand der Zellen zu vermeiden. Es hat sich gezeigt, dass gerade die Eingriffe zur Überführung von Zellen von der zweidimensionalen Anordnung in eine dreidimensionale Anordnung oder umgekehrt zu erheblichen Beeinträchtigungen, Schädigungen oder Stresssituationen führen, durch die die Zellen in einen Zustand geraten, der mit der in vivo Situation nur noch wenig zu tun hat. Besonders problematisch ist hierbei die Proteasenbehandlung (z.B. Trypsinisierung) zum Ablösen der Zellen von ihrer Oberfläche. Außerdem werden bei der Behandlung mit einer Protease (z.B. Trypsin) Oberflächenrezeptoren (z.B. Integrinrezeptoren) und andere Proteine der Zelloberfläche zum Teil verdaut, was u.a. erhebliche Auswirkungen auf das Signalling ins Innere der Zellen und damit auf deren Gesamtzustand hat. Ferner wird das Adhäsionsverhalten der Zellen beeinträchtigt, da intakte Oberflächenproteine neu synthetisiert werden müssen. Im Gegensatz dazu verbleiben die Zellen gemäß dieser Erfindung während des gesamten Behandlungs- und Untersuchungsverfahrens in ihrem adhärenten Zustand. Dieser entspricht weitgehend den physiologischen Verhältnissen, wobei Artefakte weitgehend vermieden werden können. Andererseits können die Zellen auf einem gemeinsamen Substrat gleichzeitig, d.h. vergleichend behandelt werden und nachfolgend auch vergleichend untersucht werden.

Der Primär- und der Sekundärträger gemäß der Erfindung weist einen flächigen Grundkörper auf, der aus Glas, Metall oder Plastik (z.B. Polystyrol, Polyethylen, Plexiglas, Polycarbonat, Polyester, Teflon) bestehen kann. Glas, Polystyrol und Polyethylen sind bevorzugt. Die Stärke (Dicke) des Grundkörpers kann sehr unterschiedlich sein und wird vorzugsweise mit den anderen Elementen des erfindungsgemäßen Verfahrens, insbesondere mit dem Sekundärträger, abgestimmt. Üblicherweise beträgt die Stärke einige Millimeter. Der Grundkörper kann jedoch auch als Folie ausgebildet sein mit einer Stärke im Bereich von 0,05 bis 0,5 mm. Die Mindeststärke sollte in Abhängigkeit vom verwendeten Material so bemessen sein, dass abgetrennte Trägerabschnitte über ausrelchend Stabilität verfügen, so dass das Positionieren auf dem Sekundärträger möglich ist. Die Oberfläche des Primär- und des Sekundärträgers ermöglicht das Anhaften und adhärente Wachsen der Zellen. Zu diesem Zweck wird der Grundkörper für den Primär- und/oder den Sekundärträger auf der den Zellen zugewandten Seite vorzugsweise mit einer Beschichtung versehen, die das Anhaften und adhärente Wachsen der Zellen ermöglicht. Solche Beschichtungen und das Aufbringen solcher Beschichtungen sind Fachleuten der Zellkultur bekannt. In Frage kommen vor allem proteinartige und polysaccharidartige Beschichtungen, insbesondere solche auf der Basis von Proteinen oder Polysacchariden der extrazellulären Matrix und Derivate oder Modellverbindungen derselben. Beispiele für Beschichtungen sind verschiedene Collagene, Heparansulfat, Fibronectin, Polylysin, Laminin, Vitronectin, Entactin etc.

In Stufe (a) des erfindungsgemäßen Verfahrens werden adhärente Zellen auf dem Primärträger bereitgestellt, um (in Stufe b) eine Zellschicht auf der Oberfläche des Primärträgers wachsen zu lassen. Geeignete Bedingungen zum Kultivieren der Stufe (b), insbesondere bezüglich Temperatur, Kulturmedium, Belüften, Dauer der Kultur sind für viele Zelltypen im Stand der Technik bekannt (siehe z.B. Toni Lindl, Zell- und Gewebekultur, Spektrum Akademischer Verlag). Vorzugsweise wird Stufe (b) unter kontrollierten Bedingungen in einem Brutschrank durchgeführt. Typischerweise wird der Primärträger für die Kultivierung von Schritt (b) ferner in einem Behältnis gehalten, das ein Nährmedium enthält. Das Nährmedium steht in Kontakt mit den Zellen. Vorzugsweise werden die Zellen mit dem Nährmedium überschichtet. Details zu Nährmedien für verschiedene Zelltypen und die Art und Häufigkeit des Mediumwechsels sind im Stand der Technik bekannt. Zu dem Behältnis wird üblicherweise ein Deckel gehören, der die Zellkultur belüftbar vor Kontamination schützt.

In Stufe (c) des erfindungsgemäßen Verfahrens wird ein eine Zellschicht tragender Trägerabschnitt des in Stufe (b) erhaltenen Primärträgers abgetrennt und in Stufe (d) in einer solchen Lagebeziehung zu einer für die Adhärenz und das Wachstum der Zellen geeigneten Oberfläche eines Sekundärträgers positioniert, dass die Zellen vom Trägerabschnitt auf den Sekundärträger hinüberwachsen können. Das Abtrennen des eine Zellschicht tragenden Trägerabschnitts kann auf vielerlei Arten geschehen, z.B. durch Herausschneiden, Herausbrechen oder Herausstanzen. Dabei werden die Zellen der Zellschicht nur im Bereich der Bruchstellen geschädigt. Im Hauptbereich des Trägerabschnitts bleiben die Zellen weitgehend ungestört. Um das Abtrennen eines (oder mehrerer) Trägerabschnitte zu erleichtern, ist der Primärträger vorzugsweise in eine Vielzahl von Trägerabschnitten unterteilbar, deren jeder gemäß Stufe (d) positionierbar ist. Der Primärträger kann z.B. dadurch unterteilbar gestaltet werden, dass Trägerabschnitte, vorzugsweise rechteckige Trägerabschnitte, nebeneinander gelegt und durch einen gemeinsamen Rahmen zusammengehalten werden. Dann können einzelne Trägerabschnitte dem Verbund von Trägerabschnitten leicht entnommen werden. Der Primärträger kann auch dadurch unterteilbar gestaltet werden, dass Sollbruchstellen oder Sollbruchlinien oder ein Muster derselben am Primärträger vorgesehen sind. Die Sollbruchlinien können z.B. aus Vertiefungen oder Rillen im Primärträger bestehen. Sollbruchlinien können auf der den Zellen zugewandten oder der den Zellen abgewandten Seite des flächigen Primärträgers oder auf beiden Seiten deckungsgleich vorgesehen sein. Die Anordnung der Sollbruchlinien bestimmt Form und Größe der Trägerabschnitte. Im Fall eines rechteckigen Primärträgers kann ein Muster aus Sollbruchlinien z.B. aus parallel zu den Seiten verlaufenden Sollbruchlinien bestehen, so dass die Trägerabschnitte rechteckig sind. Jedoch kann die Anordnung der Sollbruchlinien vielfältig gestaltet und dem fraglichen Zweck angepasst werden. Wenn der Primärträger aus Plastik besteht, können Sollbruchlinien z.B. beim Gießen des Primärträgerrohlings vorgesehen werden. Alternativ zu Sollbruchstellen oder -linien kann die Form und Abmessung der Trägerabschnitte auch durch das Herausschneiden oder -stanzen festgelegt werden. Dies hat den Vorteil, dass keine vorgefertigte Unterteilung des Primärträgers nötig ist und große Flexibilität bezüglich der Trägerabschnitte besteht.

Ein in Stufe (c) abgetrennter Trägerabschnitt wird dann gemäß Stufe (d) so zu einem Sekundärträger in Beziehung gebracht, dass die Zellen vom abgetrennten Trägerabschnitt auf den Sekundärträger hinüberwachsen können. Dies kann im einfachsten Fall dadurch geschehen, dass der Trägerabschnitt auf den Sekundärträger gelegt wird. Dies erfordert, dass die Stärke des Trägerabschnitts vorzugsweise höchstens 1 mm, besser höchstens 0,5 mm beträgt, damit die Zellen hinüberwachsen können. Vorteilhafterweise ist am Sekundärträger eine für den Trägerabschnitt bestimmte Vertiefung vorgesehen, damit die Zellen zum Hinüberwachsen auf die Oberfläche des Sekundärträgers keine oder nur eine geringe Stufe überbrücken müssen. Ein zwischen dem in die Vertiefung eingesetzten Trägerabschnitt und dem Sekundärträger gebildeter Spalt sollte klein genug sein, damit das Hinüberwachsen der Zellen nur wenig oder gar nicht behindert wird. Ein solcher Spalt sollte vorzugsweise höchstens 0.5 mm breit sein. Alternativ kann eine Stufe und/oder ein Spalt zwischen dem Trägerabschnitt und dem Sekundärträger dadurch klein gehalten werden, dass der Primärträger und damit die Trägerabschnitte sehr dünn sind und zum Beispiel als Folie gestaltet sind. In diesem Fall brauch der Trägerabschnitt lediglich an einer gewünschten Stelle auf den Sekundärträger gelegt zu werden, was eine vorgefertigte Vertiefung auf dem Sekundärträger überflüssig macht. Es ist jedoch empfehlenswert, dass ein auf den Sekundärträger gelegter Trägerabschnitt in einer gewünschten Position fixiert wird, um ein Verrutschen zu vermeiden. Dies erleichtert die Bestimmung des Hinüberwachsens der Zellen auf den Sekundärträger. Nach dem Positionieren eines (oder mehrerer) Trägerabschnitte auf einem (oder auf mehreren) Sekundärträgern wird der Sekundärträger für das Wachstum der Zellen geeigneten Bedingungen ausgesetzt, wie z.B. in ein Behältnis mit Nährmedium gesetzt. Ferner wird auf das Kultivieren von Stufe (b) verwiesen.
Mehrere Sekundärträger mit gleichen oder verschiedenen Oberflächen können auf einer gemeinsamen Unterlage (Substrat) vorliegen und/oder zu einer Einheit verbunden sein, d.h. die mehreren Sekundärträger sind mechanisch verbunden. Die Unterlage kann aus demselben Material wie der Grundkörper bestehen. Mehrere Sekundärträger auf einem gemeinsamen Substrat können auch dadurch geschaffen werden, dass auf einem flächigen Grundkörper mehrere Bereiche mit einer Oberflächenbeschichtung aufgebracht werden, wobei die Oberflächenbeschichtung das adhärente Wachsen der Zellen ermöglicht. Die Oberflächenbeschichtungen der Bereiche können gleichartig oder verschieden sein. Die Bereiche mit Oberflächenbeschichtung können durch Bereichen ohne Oberflächenbeschichtung voneinander getrennt sein (z.B. durch Zwischenwände), so dass die Zellen auf den Bereichen mit Oberflächenbeschichtung nicht ineinander wachsen können.

Das erfindungsgemäße Verfahren kann im einfachsten Fall zum Passagieren oder Splitting der Zellen verwendet werden. Dabei wird im Vergleich zum herkömmlichen Passagieren die Überführung der adhärenten Zellen in die Suspension mit Hilfe einer Proteasenbehandlung (Trypsinisierung) vermieden, so dass eine Zellschädigung weitgehend unterbleibt. In besonders wichtigen Anwendungen der Erfindung wird das Hinüberwachsen der Zellen in Stufe (d) verfolgt, um beispielsweise die Wirkung einer Testsubstanz auf das Wachstum der Zellen zu ermitteln, um das Wachstum verschiedener Zelltypen vergleichen zu können, oder um den Einfluss verschiedener Oberflächenmaterialien des Sekundärträgers auf das Zellwachstum zu bestimmen. Dabei ist es bequem möglich, parallel Kontrollversuche (z.B. in An- und Abwesenheit einer Testsubstanz) durchzuführen, um aussagekräftige Ergebnisse zu erhalten. Insbesondere können Ergebnisse erhalten werden, die für den der in vivo Situation nahe kommenden Normalzustand der Zellen relevant sind. Im Gegensatz dazu werden im Stand der Technik vor allem Ergebnisse erhalten, die lediglich für den künstlichen Stresszustand der Zellen infolge der Proteasenbehandlung und anderer Manipulationen relevant sind.

Das erfindungsgemäße Verfahren kann manuell im kleinen Maßstab durchgeführt werden, um die Vorteile der Erfindung auszunutzen. Das erfindungsgemäße Verfahren kann jedoch auch automatisiert werden, um im großen Maßstab mit hohem Durchsatz durchgeführt zu werden. Zum Beispiel kann die Wirkung einer Vielzahl von Testsubstanzen oder einer Vielzahl von Konzentrationen einer Vielzahl von Testsubstanzen auf einen oder auf mehrere Zelltypen untersucht werden. Dies kann im Rahmen von Toxizitätsstudien oder im Rahmen der Wirkstoffsuche sein, wobei in beiden Fällen die Aussagekraft der Zell-Assays durch die erfindungsgemäßen Vorteile im Vergleich zu Zell-Assays des Standes der Technik stark erhöht ist. Ein weiterer wichtiger Vorteil der Erfindung liegt darin, dass durch die stark erhöhte Aussagekraft die Zahl von Tierversuchen massiv reduziert werden kann. Tierversuche sind im Stand der Technik unter anderem gerade deswegen in großem Maßstab notwendig, da die Aussagekraft von in vitro Tests auf der Ebene der Zellkultur bislang beschränkt ist.

Für die obengenannten Untersuchungen wird das Hinüberwachsen der Zellen von einem Trägerabschnitt auf den Sekundärträger und/oder die Ausbreitung der Zellen auf dem Sekundärträger und/oder die Beschaffenheit der sich auf einem Sekundärträger ausbreitenden Zellen bestimmt. Eine besonders wichtige Untersuchungsmethode hierfür ist die Lichtmikroskopie. In einer Ausgestaltung der Erfindung ist der Primärträger und/oder der Sekundärträger durchsichtig oder durchscheinend, so dass bei Mikroskopierung von oben eine Beleuchtung von unten möglich ist. Für die Mikroskopierung ist der Sekundärträger vorzugsweise ausreichend dünn (ca. 1 mm oder weniger), um die Bildqualität nicht zu beeinträchtigen. Dies gilt insbesondere für die konfokale Fluoreszenzmikroskopie.

Durch Mikroskopie kann die Geschwindigkeit des Hinüberwachsens der Zellen auf den Sekundärträger sowie der Zustand der Zellen verfolgt und ausgewertet werden. Zur Bestimmung der Geschwindigkeit können Markierungen auf dem Sekundärträger nützlich sein, die in regelmäßigen Abstandsintervallen von einem Trägerabschnitt vorgesehen sein können. Für automatisierte Ausführungsformen des erfindungsgemäßen Verfahrens ist es besonders vorteilhaft, wenn die Mikroskopie mit automatisierten, computergestützten Verfahren zur Auswertung der mikroskopischen Bilder kombiniert wird. Insbesondere das Fortschreiten des Zellwachstums auf dem Sekundärträger kann durch regelmäßiges automatisiertes Mikroskopieren zusammen mit geeigneter Software zur Bildauswertung leicht verfolgt und die anfallenden Daten elektronisch gespeichert und analysiert werden.

Verschiedene Mikroskopiemethoden sind mit dem Verfahren der Erfindung kombinierbar. Dazu gehört in erster Linie die Lichtmikroskopie. Die Beleuchtung kann dabei von oben oder von unten stattfinden. Ferner ist das erfindungsgemäße Verfahren zur Behandlung von Zellkulturen mit der Fluoreszenz-Mikroskopie kombinierbar, die im Stand der Technik bekannt ist. Für die Fluoreszenz-Mikroskopie wird der Sekundärträger vorzugsweise in einer schwarzen oder dunklen, von mindestens einer Seite zugänglichen Box untersucht, um die Probe abzudunkeln. Form und Größe dieser Box werden vorzugsweise auf die Form und die Größe der Sekundärträger abgestimmt. Alternativ kann in einer Dunkelkammer gearbeitet werden. Die Fluoreszenz-Mikroskopie erweitert die Anzahl beobachtbarer Phänotypen der auf dem Sekundärträger wachsenden Zellen. Z.B. erlaubt das Labelling von Zellorganellen oder Zellstrukturen mit Antikörpern, die fluoreszierend markiert sind, die Beobachtung dieser Organellen in Abhängigkeit von Testsubstanzen oder von der Beschaffenheit der Sekundärträgeroberfläche. Beispiele von Zellorganellen oder Zellstrukturen, die mittels Fluoreszenz-Mikroskopie beobachtbar sind, sind Mitochondrien, das endoplasmatische Retikulum (ER), der Golgi-Apparat, Lysosomen, Endosomen, Zellkern und dessen Membran (Kernmembran), der Zellmembran, Zytoskelett etc. Verfahren zum Sichtbarmachen solcher Zellorganellen oder Zellstrukturen mit fluoreszenzmarkierten Antikörpern sind heute Standardverfahren. Für intrazelluläre Strukturen werden die zu untersuchenden Zellen dabei üblicherweise permeabilisiert, um das Eintreten der Antikörper in die Zellen zu ermöglichen.

Neben der Geschwindigkeit des Hinüberwachsens und der Ausbreitung der Zellen auf dem Sekundärträger können viele weiter wichtige Phänotypen der Zellen bestimmt und ausgewertet werden. Dazu gehört das Auftreten von Nekrose oder Apoptose, die Zellteilungsrate, das Adhäsionsverhalten (auch in Abhängigkeit der Oberfläche des Sekundärträgers), Zellbewegungsphänomene (z.B. initiale Retraktion), Zell-Zell-Erkennung, Membranpotential, Struktur der Plasmamembran usw. Diese Phänotpyen werden insbesondere in An- und Abwesenheit von Testsubstanzen untersucht. Testsubstanzen können Metallionen, Anionen oder organische Verbindungen sein. Solche organischen Verbindungen können potentielle pharmazeutische Wirkstoffe sein oder Verbindungen, deren Toxizität ermittelt werde soll. Insbesondere bei der Untersuchung von Testsubstanzen werden vorzugsweise mehrere Konzentrationen der Testsubstanz untersucht.

In einer wichtigen Ausführungsform der Erfindung wird die Wirkung einer Testsubstanz auf die Zellen untersucht. Hierfür werden mindestens zwei Trägerabschnitte des Primärträgers in Stufe (c) abgetrennt und in Stufe (d) auf verschiedene Sekundärträger positioniert. Vorzugsweise sind die Oberflächen der verschiedenen Sekundärträger gleichartig, d.h. sie wurden mit demselben Beschichtungsmaterial auf vergleichbare Weise beschichtet. Dann kann das Hinüberwachsen der Zellen und ihre Ausbreitung auf den Sekundärträgern in Anwesenheit (Test) und in Abwesenheit (Kontrolle) der Testsubstanz vergleichend verfolgt werden. Die Herkunft der mindestens zwei Trägerabschnitte von demselben Primärträger ist bevorzugt, da die Ausgangssituation für den Test und die Negativkontrolle dann weitestgehend übereinstimmt. Typischerweise wird die Testsubstanz dem Medium für die Zellen des Sekundärträgers beigemischt. Es ist besonders bevorzugt, mehrere Konzentrationen der Testsubstanz zu testen (z.B. für Toxizitätsuntersuchungen), wobei die weiteren benötigten Trägerabschnitte im Idealfall von demselben Primärträger abgetrennt wurden. Um ein möglichst umfassendes Bild von der Wirkung der Testsubstanz zu erhalten, kann sie parallel oder nacheinander mit verschiedenen Zellen, die aus verschiedenen Geweben stammen, getestet werden. Weiterhin kann die Wirkung einer Testsubstanz auf Zellen aus gesundem Gewebe und aus krankem Gewebe (z.B. Krebszelllinien) untersucht und verglichen werden. So kann ein umfassendes Bild der Wirkung einer Testsubstanz auf Zellen einer Vielzahl von Geweben gewonnen werden, wodurch dieses Verfahren Tierversuchen oder klinischen Versuchen überlegen ist, da bei diesen in vivo Versuchen die Wirkung auf verschiedene Gewebe oder Organe in der Regel nicht möglich ist.

In einer wichtigen Ausführungsform werden die Stufen (a) bis (c) mit zwei oder mehr Zelltypen getrennt auf verschiedenen Primärträgern durchgeführt, und Trägerabschnitte mit Zellschichten verschiedener Zelltypen werden in Stufe (d) auf
(i) verschiedene Positionen eines Sekundärträgers oder
(ii) äquivalenten Positionen verschiedener Sekundärträger mit gleichartigen Oberflächen positioniert. Die zwei oder mehr Zelltypen können sich beispielsweise in einem oder mehreren der folgenden Punkte unterscheiden: Alter der Spender der Zellen, Geschlecht der Spender der Zellen, genetische Disposition der Spender der Zellen bezüglich einer Krankheit und Herkunft der Zellen aus bestimmten Gewebetypen. Klinische Tests mit potentiellen Wirkstoffen werden häufig mit bestimmten Patientengruppen durchgeführt, beispielsweise mit Männern einer bestimmten Altersklasse. Bei der Auswertung werden die individuellen Ergebnisse gemittelt,
wobei die individuelle genotypabhängige Information verloren geht. Ferner werden die erhaltenen Ergebnisse auf andere Patientengruppen, wie z.B. Frauen, Kinder oder alte Menschen extrapoliert. Das erfindungsgemäße Verfahren erlaubt auf einfache Weise, verschiedene Zelltypen, die z.B. aus den gleichen Geweben verschiedener Individuen stammen, miteinander zu vergleichen. Insbesondere können altersspezifische oder geschlechtsspezifische Unterschiede auf diese Weise ermittelt werden. Ferner können Individuen identifiziert werden, die aufgrund ihrer genetischen Disposition auf einen bestimmten Wirkstoff nicht ansprechen. Darüber hinaus kann für einen bestimmten Patienten der für ihn wirksamste oder nebenwirkungsfreiste Wirkstoff von mehreren verfügbaren Wirkstoffen ermittelt werden. Die Erfindung ist daher ein wichtiger Bestandteil der immer wichtiger werdenden individuellen Medizin, bei der für einen bestimmten Patienten bei einer bestimmten Indikation das geeignetste von mehreren verfügbaren Medikamenten bestimmt wird.

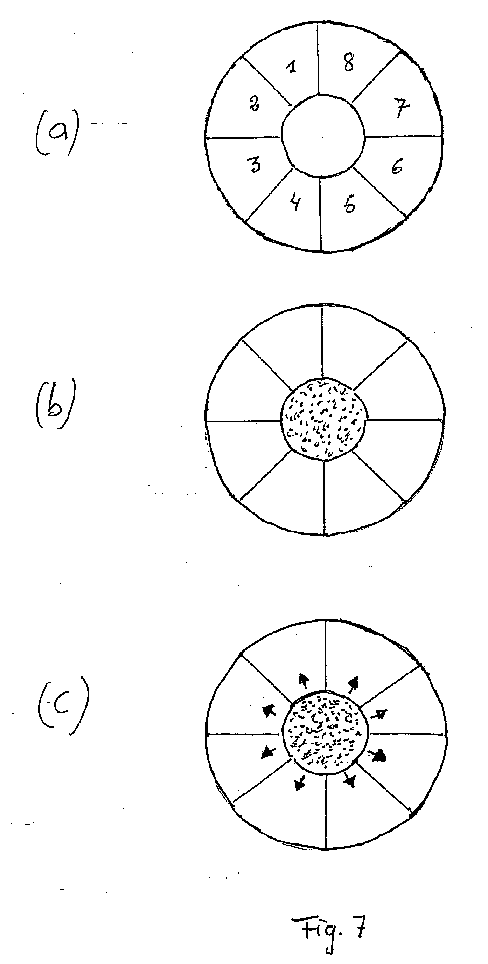

Bei einer dritten bevorzugten Ausgestaltung der Erfindung sind mehrere, vorzugsweise streifenförmige oder sektorförmige, Sekundärträger, deren Oberflächen (oder Oberflächenbeschichtungen) sich bei Bedarf unterscheiden können, auf einem gemeinsamen Substrat so angeordnet, dass jeder sektorförmige Sekundärträger an den positionierten Trägerabschnitt angrenzt (siehe Figuren 7 bis 10). Die Zellen des positionierten Trägerabschnitts können dann auf die verschiedenen Sekundärträger hinüberwachsen, wobei sich die Geschwindigkeit des Hinüberwachsens und der Ausbreitung auf den verschiedenen Sekundärträgern in Abhängigkeit von der Oberflächenbeschaffenheit der Sekundärträger unterscheiden kann. Dies kann dazu benutzt werden, für einen bestimmten Zelltyp und unter bestimmten Bedingungen geeignete Oberflächen zu finden und aus einer Vielzahl vorhandener Oberflächen auszuwählen bzw. danach zu screenen. Bei einem streifenförmigen Sekundärträger besteht der große Vorteil, dass die von einem Ende des streifenförmigen Sekundärträgers aus wachsenden Zellen sich in einer vorgegebenen Richtung fortpflanzen, so dass näher am Trägerabschnitt befindliche Zellen einem früheren Entwicklungsstadium entsprechen und weiter vom Trägerabschnitt entfernte Zellen einem späteren Entwicklungszustand. Vergleichende Untersuchungen können sich auf den Vergleich verschiedener Zellinien beziehen oder auf den Vergleich verschiedener Bedingungen, z.B. die Gegenwart von Wirkstoffen, oder auf den Vergleich verschiedener Oberflächenbehandlungen des Grundkörpers für das adhärente Wachsen der Zellen.

Das erfindungsgemäße Verfahren zur Behandlung von Zellkulturen kann mit allen adhärenten Zellen durchgeführt werden. Dazu gehören adhärente prokaryotische Zellen und eukaryotische Zellen. Eukaryotische Zellen sind bevorzugt. Bevorzugte eukaryotische Zellen sind adhärente tierische und pflanzliche Zellen, wobei tierische Zellen bevorzugt sind. Besonders bevorzugt sind Zellen von Säugetieren und insbesondere humane Zellen. Diese Zellen können gesunde Zellen oder kranke Zellen (z.B. Krebszelllinien) sein. Die Zellen können aus jedwedem Gewebe stammen, sofern sie adhärent sind. Beispiele für solche Zellen sind Epithelzellen, Fibroblasten, Endothelzellen (z.B. HUVEC-Zellen), Hepatozyten, neuronale Zellen, Osteoklasten, CHO (chinese hamster ovary)-Zellen usw. Die Zellen können auch rekombinante oder transformierte Zellen sein.

Diese Erfindung stellt ferner ein System zur Durchführung des erfindungsgemäßen Verfahrens bereit, das umfasst:
(a) einen Primärträger mit abtrennbaren Trägerabschnitten für adhärente Zellen, wobei der Primärträger in einem Behältnis angeordnet ist, das eine Kultivierung von Zellen auf einer Oberfläche des Primärträgers erlaubt;
(b) einen Sekundärträger, der zur Aufnahme und Fixierung eines Trägerabschnitts geeignet ist, so dass ein Hinüberwachsen von Zellen von einer Oberfläche des Trägerabschnitts auf eine benachbarte Oberfläche des Sekundärträgers durch den Übergangsbereich zwischen Trägerabschnitt und Sekundärträger im wesentlichen nicht behindert wird.

Der Primärträger und der Sekundärträger sind vorzugsweise aufeinander abgestimmt. Zur Aufnahme eines Trägerabschnitts kann der Sekundärträger eine Vertiefung aufweisen, die den Abmessungen des Trägerabschnitts entspricht. Die Aufnahme des Trägerabschnitts in eine Vertiefung ist gleichzeitig zu seiner Fixierung geeignet. Wird z.B. im Fall eines Trägerabschnitts in Form einer Folie dieser auf den Sekundärträger gelegt, ist eine anders geartete Fixierung des Trägerabschnitts vorzugsweise erforderlich, damit das Hinüberwachsen und/oder eine Ausbreitung der Zellen bestimmt werden kann, ohne dass infolge eines Verrutschens des Trägerabschnitts die Bestimmung behindert oder unmöglich gemacht wird. Ob das Hinüberwachsen der Zellen durch den Übergangsbereich im wesentlichen behindert wird, ist abhängig von der Art des Trägerabschnitts, der Art der Aufnahme und Fixierung des Trägerabschnitts auf dem Sekundärträger, der Art der Zellen, den Oberflächen von Trägerabschnitt und Sekundärträger sowie von den sonstigen (Kultur-) Bedingungen (z.B. Anwesenheit einer Testsubstanz im Kulturmedium). Ob eine wesentliche Behinderung vorliegt, kann durch Kontrollexperimente bestimmt werden. Hierbei wird beispielsweise das Hinüberwachsen unter gegebenen Bedingungen mit einer für das Hinüberwachsen optimalen Situation (z.B. keine Stufe und kein Spalt zwischen Trägerabschnitt und Sekundärträger, übereinstimmende und für den fraglichen Zelltyp optimale Oberflächenbeschichtung etc.) verglichen.
Weitere Ausgestaltungen des Systems können gemäß der Beschreibung und den Figuren vorgenommen werden. Zum Beispiel ist vorzugsweise für den Primär- und/oder den Sekundärträger ein Behältnis zur Aufnahme von Medium vorgesehen, um die Zellen mit dem Medium zu kontaktieren. Ferner können mehrere insbesondere streifen- oder sektorförmige Sekundärträger auf einem gemeinsamen Substrat (Unterlage) angeordnet sein. Auf dem Sekundärträger können Markierungen vorgesehen sein, die das Bestimmen der Ausbreitung (oder der Geschwindigkeit der Ausbreitung) der Zellen auf dem Sekundärträger erleichtern. Diese Markierungen können zum Beispiel im Abstand von 1 Millimeter oder mehreren Millimetern angebracht sein. Besteht der Sekundärträger aus transparentem Plastik, können die Markierungen beispielsweise in den für den Spritzguss verwendeten Formen als Vertiefungen vorgesehen sein, so dass die Markierungen als linienförmige Materialauflagen z.B. auf der Unterseite des Sekundärträgers erhalten werden.

### BESCHREIBUNG DER FIGUREN

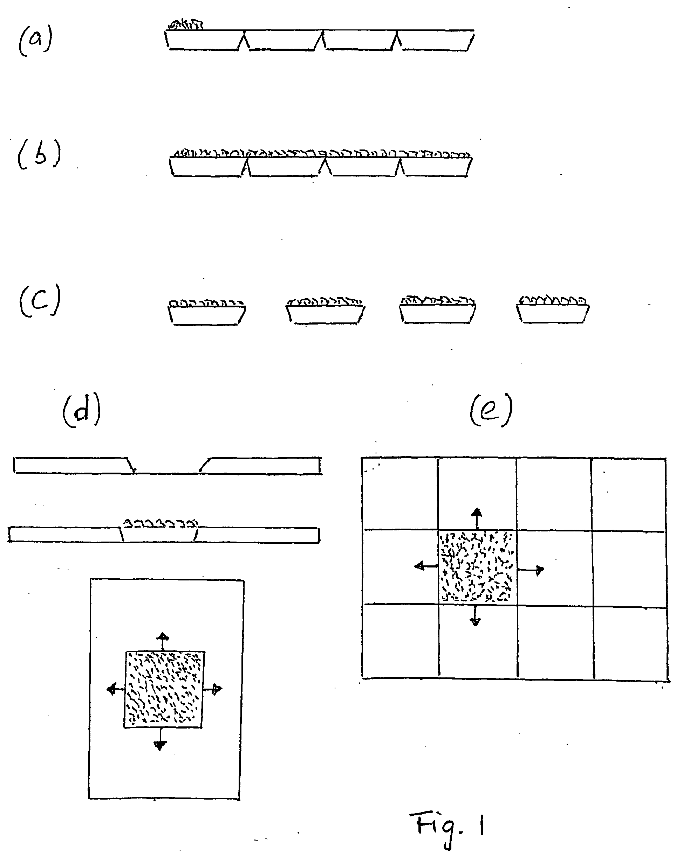

Fig. 1 gibt einen schematischen Überblick über die Verfahrenschritte des erfindungsgemäßen Verfahrens. Die Trägerabschnitte der Primärkultur sind an Sollbruchstellen durch mechanische Einwirkung leicht voneinander trennbar. (a) zeigt einen aus vier Trägerabschnitten bestehenden Primärträger mit einer Probe adhärenter Zellen, die links aufgebracht wurde und sich als Primärkultur nach rechts ausbreitet. (b) zeigt eine auf der Oberfläche des Primärträgers ausgebildete konfluente Zellschicht (Monolayer). In (c) wurden die eine Zellschicht tragenden Trägerabschnitte voneinander abgetrennt. (d) zeigt (oben) einen Sekundärträger mit einer Vertiefung zur Aufnahme eines Trägerabschnitts. Darunter wurde ein Trägerabschnitt mit einer Zellschicht so in dem Sekundärträger positioniert, dass die Zellen auf den Sekundärträger hinüberwachsen können. (d) zeigt unten eine Aufsicht auf einen Sekundärträger, der einen positionierten Trägerabschnitt enthält. Die Pfeile geben die Richtungen an, in die die Zellen auf den Sekundärträger hinüberwachsen können. Dieses Hinüberwachsen kann in Anwesenheit und/oder in Abwesenheit einer Testsubstanz verfolgt werden. (e) zeigt einen anders gearteten Sekundärträger, der einen positionierten Trägerabschnitt enthält. Dieser Sekundärträger (e) kann z.B. zum Erhalt der Zellkultur dienen durch stressfreies Passagieren der Zellkultur.

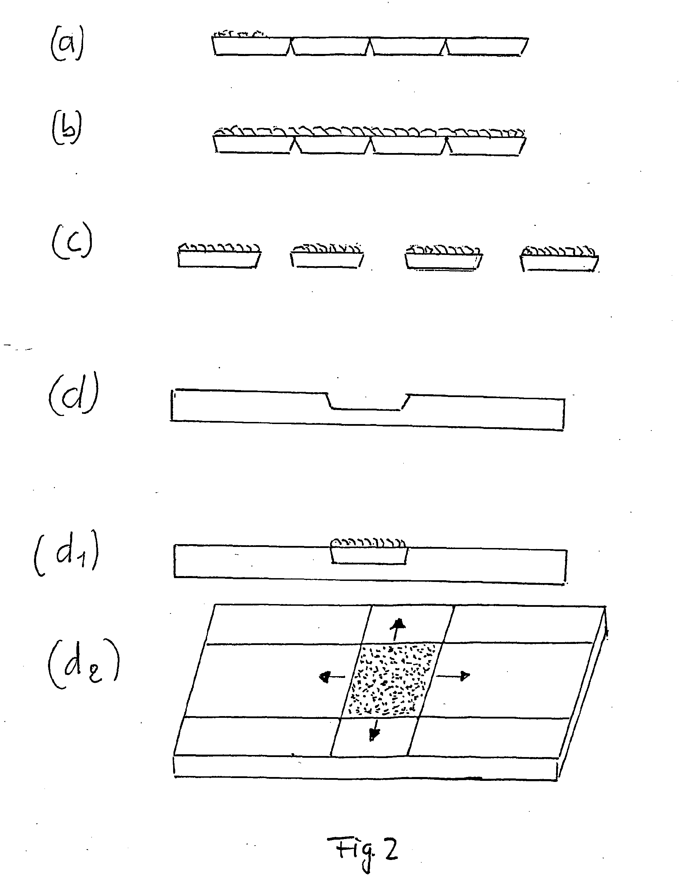

Fig. 2 zeigt in (d1 und d2) mehrere Sekundärträger auf einem gemeinsamen Substrat. Die Zellen können auf vier verschiedene Sekundärträger (mit Pfeilen gekennzeichnet) hinüberwachsen, da jeder der vier Sekundärträger an den positionierten Trägerabschnitt angrenzt. (a) bis (c) entsprechen denen von Fig. 1.

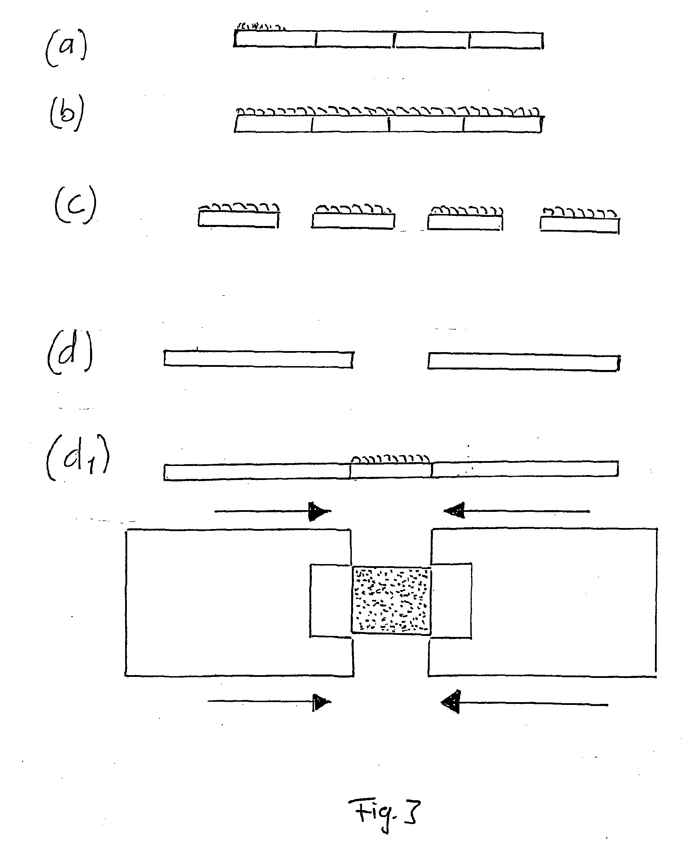

Fig. 3 zeigt in (d) einen zweiteiligen Sekundärträger. Der Trägerabschnitt wird durch Zusammenschieben der Hälften des Sekundärträgers und Einschließen des Trägerabschnitts positioniert (d1). Hierfür weisen die Hälften des Sekundärträgers vorzugsweise eine Nut auf, in die Schienen des Trägerabschnitts eingreifen können, damit der Trägerabschnitt in der gewünschten Position fixiert wird (nicht dargestellt).

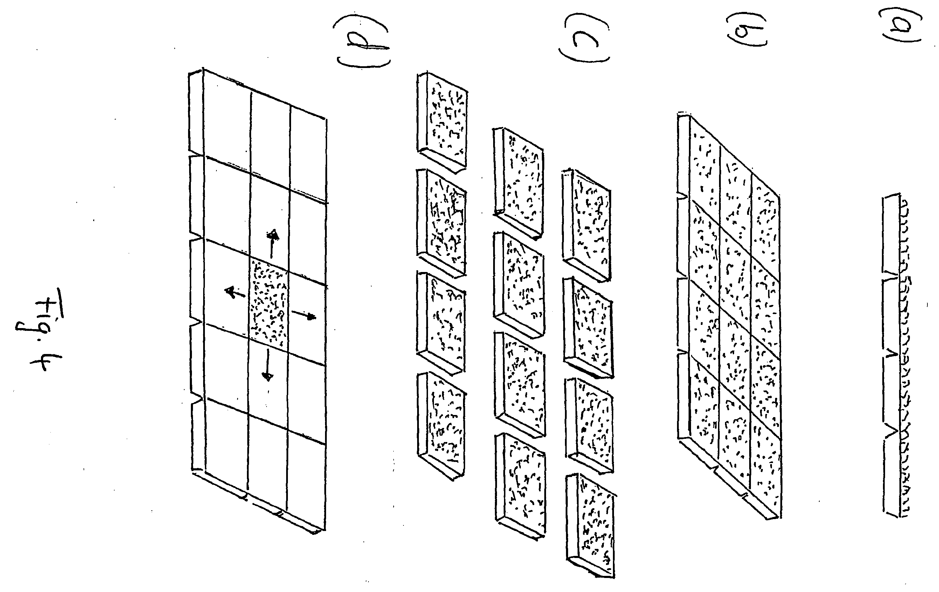

Fig. 4 veranschaulicht die Dauerkultur von adhärenten Zellen gemäß des Verfahrens der Erfindung durch stressfreies Passagieren (Subkultivieren) der Zellen. (b) zeigt eine konfluente Zellschicht auf einem Primärträger. In (c) wurde der Primärträger in Trägerabschnitte zerlegt. Jeder dieser eine Zellschicht tragenden Trägerabschnitte kann auf einem Sekundärträger positioniert werden (d), wodurch die Zellkultur passagiert (subkultiviert) wird. Der Sekundärträger (d) ist wie der Primärträger mittels Sollbruchlinien in Trägerabschnitte unterteilbar. Dadurch kann nach Ausbilden einer Zellschicht auf dem Sekundärträger erneut ein oder mehrere Trägerabschnitte abgetrennt und die Zellschicht erneut passagiert werden.

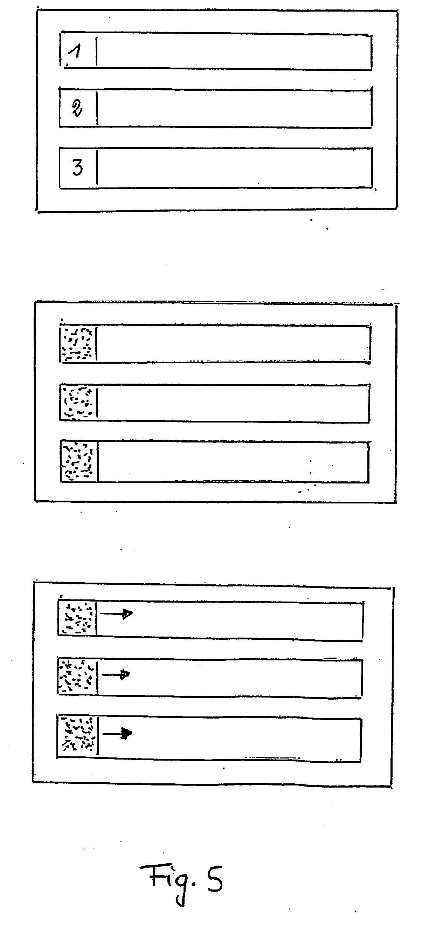

Fig. 5 zeigt schematisch drei streifenförmige Sekundärträger. Die Sekundärträger können wie gezeigt auf einem gemeinsamen Substrat vorliegen. Am linken Ende der Streifen ist auf jedem Sekundärträger eine Position für einen Trägerabschnitt vorgesehen. Mitte: am linken Ende jedes Streifens wurde ein Trägerabschnitt positioniert. Die Zellen können sich in Richtung der Pfeile ausbreiten und auf die Sekundärträger hinüberwachsen. Die Anordnung der Steifen erlaubt dabei ein einfaches Vergleichen der Zellausbreitung auf den drei Sekundärträgern. Die verschiedenen Sekundärträger können sich anhand ihrer Oberfläche unterscheiden, um die Ausbreitung der Zellen auf verschiedenen Oberflächen der Sekundärträger zu verfolgen. Alternativ können sich die Oberflächen der Sekundärträger gleichen. Vorzugsweise sind dann Trennwände zwischen den Sekundärträgern vorgesehen (nicht gezeigt), die es erlauben, die Zellen auf den Sekundärträgern unterschiedlichen Medien auszusetzen. Beispielsweise kann die Ausbreitung der Zellen in Abwesenheit und in Anwesenheit zweier Konzentrationen einer Testsubstanz (oder zweier verschiedener Testsubstanzen) verglichen werden. Alternativ zum Vorliegen der Sekundärträger auf einem gemeinsamen Substrat können die Sekundärträger getrennt voneinander auf ihren Grundkörpern vorliegen (nicht gezeigt). Dann können die Zellen auf jedem Sekundärträger in einem eigenen Behältnis mit Medium (mit oder ohne Testsubstanz) behandelt werden ohne dass die genannten Trennwände nötig sind.

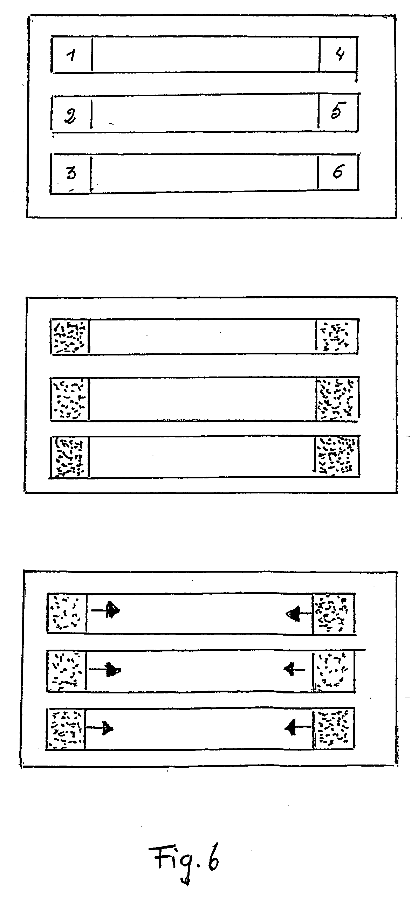

Fig. 6 zeigt eine weitere Ausgestaltung streifenförmiger Sekundärträger auf einem gemeinsamen Substrat, wobei im Unterschied zu denen von Fig. 5 an beiden Enden jeden Streifens eine Position für einen Trägerabschnitt mit einer Zellschicht vorgesehen ist. Die Zellen der an den beiden Enden eines Sekundärträgers positionierten Trägerabschnitte können (i) die gleichen sein (z.B. von demselben Primärträger stammen) oder (ii) verschieden sein. Im ersteren Fall können parallel zwei Experimente mit den gleichen Zellen auf einem Streifen (Sekundärträger) durchgeführt werden, was Aussagen zur Reproduzierbarkeit der Experimente erlaubt und so die Aussagekraft erhöht. Im zweiten Fall kann die Zellausbreitung auf einem Streifen (Sekundärträger) für zwei unterschiedliche Zelltypen unter sonst gleichen Bedingungen verfolgt werden. Ferner kann beim Aufeinandertreffen der beiden Zelltypen auf dem Streifen die Affinität und Verträglichkeit dieser Zelltypen zueinander verfolgt werden. Daneben oder zusätzlich sind die zu Fig. 5 genannten Verwendungen durchführbar. Wie in der Beschreibung zu Fig. 5 beschrieben können Sekundärträger dieser Art auch getrennt voneinander ohne gemeinsames Substrat vorliegen.
Fig. 7 zeigt in (a) mehrere sektorförmige Sekundärträgern auf einem gemeinsamen Substrat. Zentral ist eine Position für einen runden Trägerabschnitt vorgesehen. In (b) wurde ein solcher runder Trägerabschnitt zentral positioniert. Die Zellen können in Pfeilrichtung auf die verschiedenen Sekundärträger hinüberwachsen. Die abgerundete Form der Sekundärträger zum zentralen Bereich hin sorgt für ein enges Angrenzen an den Trägerabschnitt. Die Anwendungsmöglichkeiten einer solchen Anordnung entsprechen weitgehend derjenigen von Fig. 5.

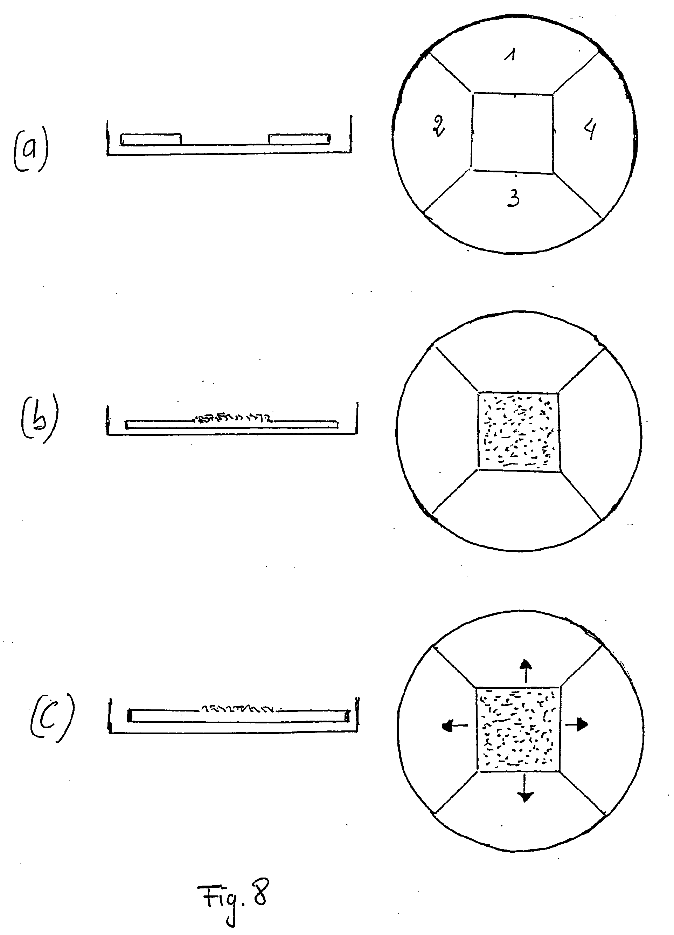

Fig. 8 zeigt ein Substrat mit einer weiteren Anordnung von Sekundärträgern in Seitenansicht (links) und in Aufsicht (rechts). Zentral im Substrat ist eine Position für einen quadratischen Trägerabschnitt vorgesehen. Nach dem Positionieren des Trägerabschnitts grenzt dieser an vier Sekundärträger an, so dass die Zellen auf diese hinüberwachsen können. Die Seitenansichten (links) zeigen ferner ein Behältnis zur Aufnahme des Substrats.

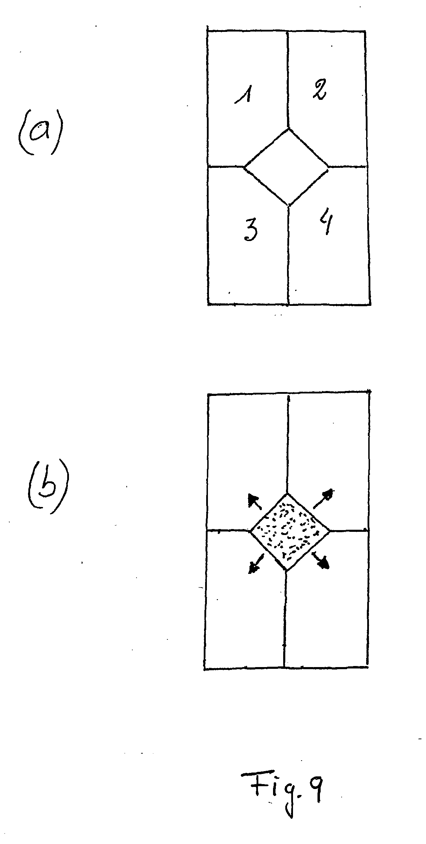

Fig. 9 zeigt ein Substrat mit einer weiteren Anordnung von Sekundärträgern. Zentral ist eine Position für einen quadratischen Trägerabschnitt vorgesehen. Nach dem Positionieren des Trägerabschnitts grenzt dieser an vier Sekundärträger an, so dass die Zellen auf diese hinüberwachsen können.

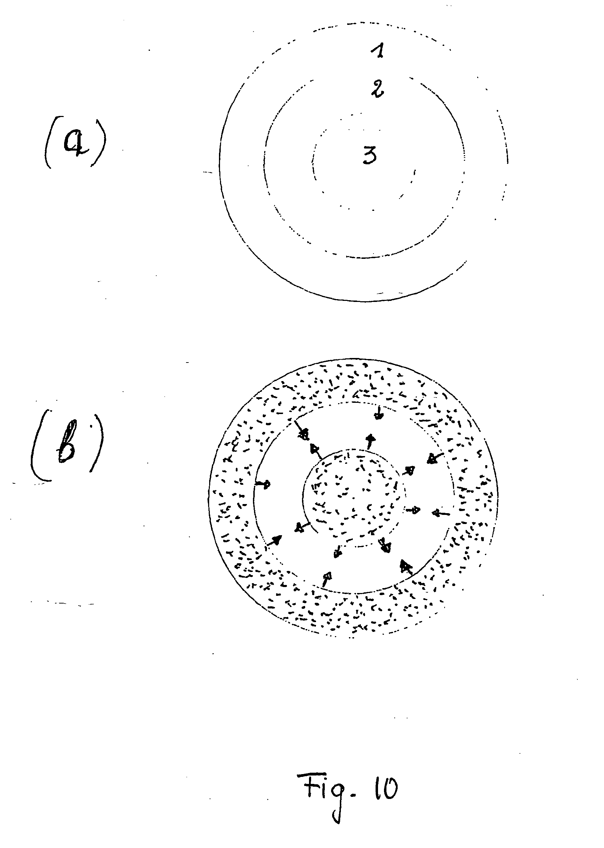

Fig. 10 zeigt ein Substrat mit einer kreisförmigen Anordnung eines Sekundärträgers (Bereich 2). Bereich 1 und 3 sind für einen ringförmigen bzw. einen kreisförmigen Trägerabschnitt vorgesehen. Die Zellen können dann auf den Bereich 2 hinüberwachsen.

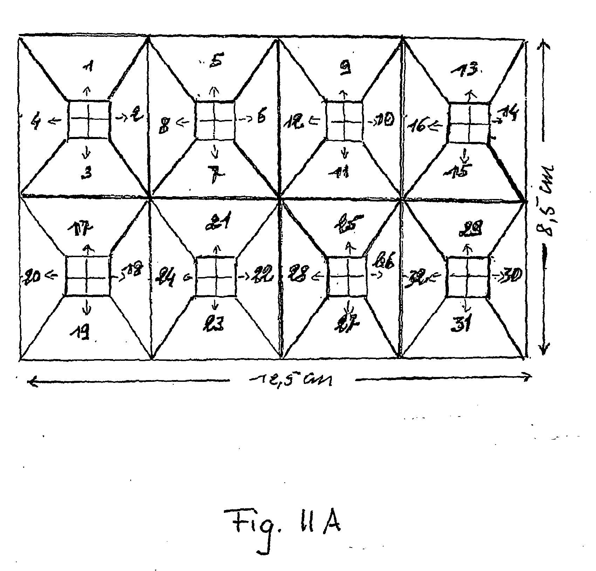

Fig. 11A zeigt eine Aufsicht auf eine Einheit aus acht durch Trennwände voneinander getrennte Sekundärträger. Im Zentrum eines jeden Sekundärträgers befindet ein Trägerabschnitt, der von einem Primärträger abgetrennt wurde. Von jedem Trägerabschnitt aus können die Zellen auf vier verschiedene Flächen des Sekundärträgers hinüberwachsen.

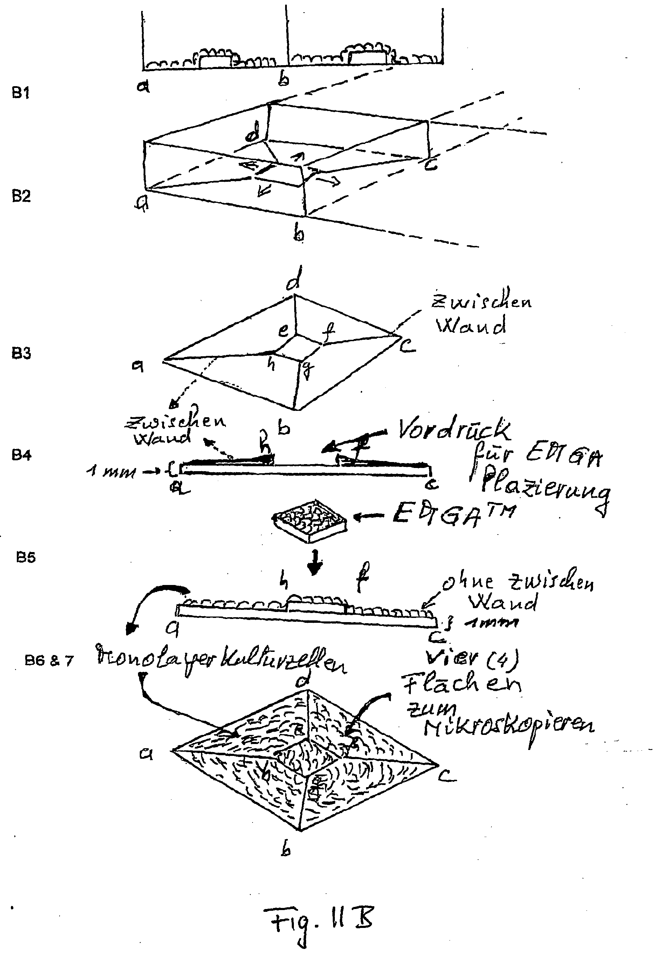

Fig. 11B zeigt den Aufbau der Sekundärträger von Fig. 11A. B1 zeigt schematisch eine Seitenansicht zweier Sekundärträger mit einer konfluenten Zellschicht. B2 ist eine perspektivische schematische Ansicht eines der Sekundärträger. B3 zeigt den Sekundärträger ohne Trennwände zu den anderen Sekundärträgern. Zwischenwände zwischen den 4 verschiedenen Flächen, auf die die Zellen hinüberwachsen können, sind angedeutet. Die mit a, b, c und d bezeichneten Ecken entsprechen den mit a, b, c bzw. d bezeichneten Ecken in den übrigen Darstellungen von Fig. 11B. B4 ist eine Seitenansicht des Sekundärträgers, der eine Dicke von 1 mm aufweisen kann. Zwischenwände sind durch dunkle Flächen angedeutet. B5 zeigt einen mit Zellen bewachsenen Trägerabschnitt. B6 und B7 zeigen den Sekundärträger, der von dem Trägerabschnitt aus mit adhärenten Zellen bewachsen ist. Dieser Sekundärträger weist vier Flächen auf, auf denen die Zellen mikroskopisch untersucht werden können.

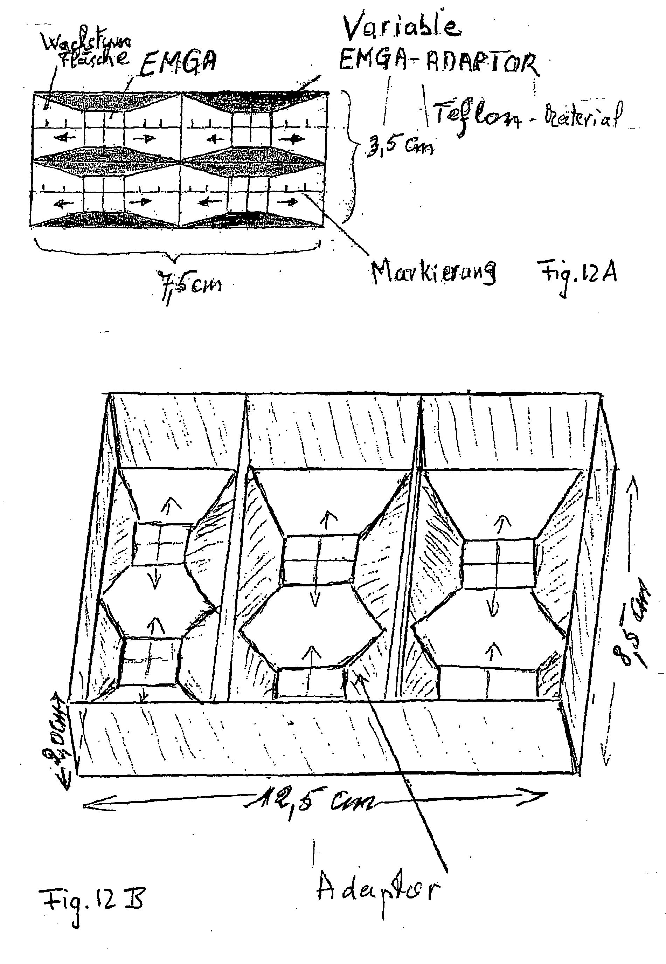

Fig. 12A zeigt 4 Sekundärträger, die zu einer (mechanischen) Einheit zusammengefasst sind. Zentral in jedem Sekundärträger kann jeweils ein Trägerabschnitt eingesetzt werden. Die Pfeile geben die Richtungen an, in die die Zellen auf den Sekundärträger hinüber wachsen können. Die dunklen Flächen deuten Adaptoren an, die zur Befestigung der Trägerabschnitte dienen. Die Trägerabschnitte können z.B. zwischen je zwei Adaptoren festgeklemmt werden. Hierzu sollten die Adaptoren eine gewisse Elastizität aufweisen. Die Adaptoren können z.B. aus Teflon sein, jedoch eignet sich hierfür eine Vielzahl an Materialien. Durch Anpassen der Adaptoren können Trägerabschnitte verschiedener Größe in einen gegebenen Sekundärträger eingesetzt werden.
Fig. 12B zeigt eine im Vergleich zu Fig. 12A anders aufgebaute und dimensionierte (mechanische) Einheit aus drei Sekundärträgern, die durch Trennwände voneinander getrennt sind. Jeder Sekundärträger erlaubt die Aufnahme von jeweils zwei Trägerabschnitten. Wiederum sind Adaptoren vorgesehen zum Festklemmen der Trägerabschnitte.

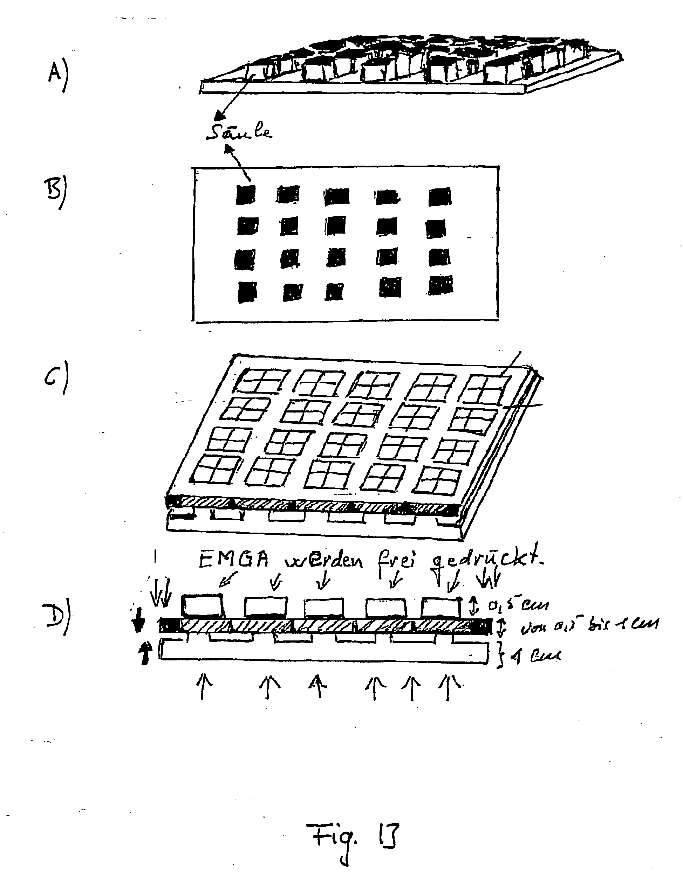

Fig. 13 zeigt eine Stanzplatte und ein Verfahren zum gleichzeitigen Abtrennen mehrerer Trägerabschnitte von einem Primärträger durch Herausstanzen der Trägerabschnitten aus dem Primärträger. Die Stanzplatte weist Erhebungen auf, deren Anordnung der Anordnung von Trägerabschnitten auf einem Primärträger angepasst sein sollte. Die Erhebungen können säulenförmig sein. A) zeigt eine perspektivische Ansicht und B) eine Aufsicht auf die Stanzplatte. Diese kann z.B. aus Metall oder aus Kunststoff (z.B. Polyethylen oder Polystyrol) bestehen. C) zeigt unten eine Stanzplatte und darüber einen Primärträger. An jeder Position eines Trägerabschnitts auf dem Primärträger weist die Stanzplatte eine Erhebung auf. Werden die Stanzplatte und der Primärträger weiter aufeinander zubewegt, drücken die Erhebungen der Stanzplatte die Trägerabschnitte aus dem Primärträger heraus. In D) befinden sich die Stanzplatte und der Primärträger so nahe beieinander, dass die Trägerabschnitte aus dem Primärträger abgetrennt wurden und auf den Erhebungen der Stanzplatte liegen. Von dort können die Trägerabschnitte für nachfolgende Arbeitsschritte, z.B. für Schritt (d) des erfindungsgemäßen Verfahrens, entnommen werden. Dies kann auch durch einen Arm eines Roboters geschehen. Die in Fig. 13 dargestellte Ausführungsform ist besonders für die Automatisierung geeignet und erlaubt einen hohen Durchsatz des erfindungsgemäßen Verfahrens. In diesem Fall werden die Stanzplatte und der Primärträger vorzugsweise automatisiert z.B. von einem Roboter aufeinander zubewegt.

### BEISPIELE

### Reagenzien

NaCl, KCI, MgCl₂, CaCl₂ (Sigma); N-2-Hydroxyethylpiperazin-N-2-ethansulfonsäure (HEPES) (Sigma); Ethylendiamin-tetraessigsäure-Dinatriumsalz (EDTA), 4,6-Diamidino-2-phenylindol (DAPI), D-Glucose (Merck, Darmstadt); Penicillin G (Sigma); Eagle's minimal essential medium (MEM) (Sigma), inactivated calf serum (abattoir-collected). Cyclohexemid (Fluka).

### Kulturmedien

HEPES - und EDTA - gepufferte Salzlösung (HBS): 1 Liter HBS wurde aus folgenden Stammlösungen hergestellt (Endkonzentrationen in Klammern): 25 ml HEPES 0,2 mol/l (5 mmol/l); 47 ml NaCl 3,0 mol/l (140 mmol/l); 5 ml KCL 0,5 mol/l (5 mmol/l); 20 ml Glucose 0,25 mol/l (5 mmol/l); 20 ml EDTA x Na₂ 10 mmol/l (0,2 mmol/l); 10 ml Penicillin mit 200 000 IE; tridestilliertes H₂O. Calzium und/oder Magnesium wurden als Chloride zugesetzt, berechnet nach den von Wolf (1972, 1973) gegebenen Formeln unter Verwendung von K_{caEDTA} = 3,8 10⁻¹¹ und K_{MgEDTA} = 2,1 . 10⁻⁹.

### Die folgenden HBS-Medien wurden verwendet:

HBS (Mg):
   Eingestellt auf mmol/l freies Mg⁺⁺, kein Zusatz von Ca⁺⁺ (enthält weniger als 10⁻⁷ mol/l freies Ca⁺⁺ aus Verunreinigungen)
HBS (a):
   Eingestellt auf 1 mmol/l freies Ca⁺⁺, kein Zusatz von Mg⁺⁺ (enthält 2,5 10⁻⁷ freies Mg⁺⁺ aus Verunreinigungen);
HBS(Ca+Mg):
   Eingestellt auf 1 mmol/l freies Ca⁺⁺ und 1 mmol/l freies Mg⁺⁺.

pH-Einstellung der Medien und pH - Kontrolle: HBS wurde durch Zusatz von kleinen Mengen 0,1 N NaOH auf die gewünschten pH-Werte eingestellt. MEM plus 10% Serum wurden vorher über Nacht bei 36°C mit dem CO₂-Gehalt der Atmosphäre äquilibriert und danach mit einer HEPES-Stammlösung auf pH 7,1 bzw. mit einem HEPES/NaOH-Puffer auf pH 7,4 oder 7,8 gebracht. Diese Lösungen enthielten 93,3 % Medium (MEM plus 10% Serum) und 18,8 mmol/l HEPES. Wenn erforderlich wurden die im biologischen Test geprüften Zusätze vorher mit HEPES oder HEPES/NaOH auf den gewünschten pH-Wert eingestellt. Die pH - Messungen wurden mit einer Glaselektrode bei 35° bis 36°C - der Temperatur, bei der biologische Tests erfolgten - durchgeführt.

### Beispiel 1

### Herstellung einer Primärkultur aus Epithel-Kapsel-Zellen des Rinderauges

Rinderaugen sind aus Schlachthöfen erhältlich. Die Augen werden im Thermosgefäß gesammelt und ins Labor transportiert. Für eine kurze Zeit (2-3 Stunden) können die Augen nach dem Tod der Tiere im Brutraum bei 35-36°C aufbewahrt werden. Dann erfolgt die Gewinnung des Epithel-Kapsel-Präparat (EKP). Hierzu wird nach dem Abtragen der Cornea etwa 2 mm vor dem Linsenäquator ein kreisrunder Schnitt gelegt. Das zentrale Epithel-Kapsel-Präparat (G₀-EKP) oder Teile davon werden auf Deckgläschen, an denen sie durch Kapillarkraft haften, gelegt, in eine Petrischale mit Kulturmedium überführt und durch kleine Glasstückchen fixiert. Für Testmaterial wurde EKP gedrittelt oder geviertelt. Die Teilpräparate wurden in kleine Petrischalen (Durchmesser = 40 oder 50 mm) mit 3 oder 4ml Medium überführt. Eine Stunde (1h) nach dem Beginn der Präparation wurde unter dem Mikroskop (Televal-Mikroskop) mit Hilfe eines kleinen Skalpells ein etwa quadratisches 1,5 bis 2,0 mm² großes Epithelareal markiert. Danach wird das übrige Epithel mit dem Skalpell vorsichtig abgeschabt. Dann erfolgt die weitere Inkubation in einer feuchten Kammer.

Zur quantitativen Auswertung der initialen Retraktion und des Spreadings (Ausbreitung) wurde die Markierung, d.h. die markierte Epithelfläche mit Hilfe eines Okularrasters zu verschiedenen Zeiten ausgemessen und die Werte auf die Startfläche bezogen, die gleich 100% gesetzt wurde. Die gesamte Prozedur, einschließlich der quantitativen Auswertung, erfolgte in einem temperierten Raum bei 35 bis 36°C (Brutraum).

### Beispiel 2

### Kultivierung und Splitten der Zellen auf Kollagen-Gel-Trägern

Die Gewinnung von säurelöslichem Kollagen erfolgte nach einem Verfahren von Michalospoulos und Pitot (Primary culture of parenchymal liver cells on collagen membranes morphological and biochemical observations: EXPRL. CELL RES. 94:70-78 (1975)) mit geringen Abänderungen (nach STEIN, G.H. und YANISSHEWSKY, R.). Rattenschwanzsehnen wurden getrocknet und in etwa 1 bis 2 cm lange Stückchen zerteilt. Zur Keimfreimachung wurde das getrocknete Material 16 Stunden mit UV-Licht im Abstand von 1 m bestrahlt.

1 g Rattenschwanzsehnen wurde mit 300 ml verdünnter Essigsäure (1:1000) versetzt und 48 Stunden bei 5 °C unter sterilen Bedingungen gerührt (Magnetrührer). Die entstandene hochviskose Lösung wurde 30 Minuten bei 16000 rpm in einer Kühlzentrifuge K 24 (Kendro), zentrifugiert. Der Überstand wurde mit verdünnter Essigsäure auf eine Proteinkonzentration von 1,4 mg pro ml eingestellt und bei 4 °C aufbewahrt.

Zur Herstellung von Kollagen-Gel wurden 1,7 ml dieser Kollagen-Lösung auf einem Träger, der in eine Petrischale (Durchmesser 50 mm) gelegt wurde, zugegeben und mit 0,4 ml einer MEM-NaOH-Lösung versetzt. Die MEM-NaOH-Lösung wurde frisch hergestellt und bestand aus 2 ml einer 10-fachkonzentrierten MEM-Lösung und 1 ml 0,34 M NaOH. Sofort nach der Zugabe der MEM-NaOH-Lösung erfolgte die Mischung mit der Kollagen-Lösung durch kreisförmige Bewegungen der Petrischalen. Nach 1 bis 5 Minuten waren die Gele fest und hafteten auf dem Glasboden des Trägers (Primär- oder Sekundärträger).

Um ein optimales Wachstum der Zellen auf den Kollagen-Gelen zu erzielen, wurden die Gele 24 Stunden vor Kulturbeginn mit serumhaltigem Medium oder HBS Medium überschichtet. Dieses Medium wurde vor der Aussaat der Zellen durch frisches Medium ersetzt. Nach dem Ausbreiten der Zellen auf dem Primärträger wurden Trägerabschnitte mechanisch vom Primärträger abgetrennt und die Zellen durch Einsetzen eines abgetrennten Trägerabschitts in einen Sekundär- oder Tertiärträger stressfrei subkultiviert. Kultivierte Zellen der Augenlinse des Rindes zeigten ein gutes Anhaften und mitotisches Wachstum auf den hier beschriebenen Kollagen-Gel Trägern.

## Patentansprüche

1. Verfahren zur Behandlung von Zellkulturen, **gekennzeichnet durch** die folgenden Stufen:
(a) Bereitstellen eines auf einer Oberfläche adhärente Zellen aufweisenden Primärträgers, wobei die Oberfläche für die Adhärenz und das Wachstum der Zellen geeignet ist;
(b) Kultivieren der Zellen auf dem Träger unter vorbestimmten Bedingungen unter Ausbildung einer Zellschicht auf der Oberfläche des Primärträgers;
(c) Abtrennung eines eine Zellschicht tragenden Trägerabschnitts des in Stufe (b) erhaltenen Trägers;
(d) Positionieren des in Stufe (c) abgetrennten Trägerabschnitts in einer solchen Lagebeziehung zu einer für die Adhärenz und das Wachstum der Zellen geeigneten Oberfläche eines Sekundärträgers, dass die Zellen vom Trägerabschnitt auf den Sekundärträger hinüberwachsen können.

2. Verfahren nach Anspruch 1, wobei der Primärträger in eine Vielzahl von Trägerabschnitten unterteilbar ist, die gemäß Stufe (d) positionierbar sind.

3. Verfahren nach einem der Ansprüche 1 oder 2, wobei für den Primärträger und/oder den Sekundärträger ein Behältnis vorgesehen ist, das ein Nährmedium oder einen Puffer für die Zellen enthält und wobei dem Nährmedium im Behältnis des Sekundärträgers eine oder mehrere Testsubstanzen zugegeben werden können.

4. Verfahren nach einem der Ansprüche 1 bis 3, wobei das Hinüberwachsen der Zellen von einem Trägerabschnitt auf den Sekundärträger und/oder die Ausbreitung der Zellen auf dem Sekundärträger und/oder die Beschaffenheit der sich auf einem Sekundärträger ausbreitenden Zellen bestimmt wird.

5. Verfahren nach einem der Ansprüche 1 bis 4, wobei in Stufe (c) zwei oder mehr Trägerabschitte des Primärträgers abgetrennt werden, die in Stufe (d) auf verschiedene Sekundärträger positioniert werden, deren Oberflächen gleichartig sind.

6. Verfahren nach einem der Ansprüche 1 bis 5, wobei das Hinüberwachsen der Zellen von einem Trägerabschnitt auf die Sekundärträger und/oder die Ausbreitung der Zellen auf den Sekundärträgern und/oder die Beschaffenheit der sich auf einem Sekundärträger ausbreitenden Zellen in Anwesenheit und in Abwesenheit einer Testsubstanz verfolgt wird, um eine Wirkung der Testsubstanz auf die Zellen zu bestimmen.

7. Verfahren nach einem der Ansprüche 1 bis 6, wobei Schritte (a) bis (c) mit zwei oder mehr Zelltypen getrennt auf jeweils einem Primärträger durchgeführt werden und Trägerabschnitte mit Zellschichten verschiedener Zelltypen auf
(a) verschiedene Positionen eines Sekundärträgers oder
(b) äquivalenten Positionen verschiedener Sekundärträger mit gleichartigen Oberflächen
positioniert werden.

8. Verfahren nach einem der Ansprüche 1 bis 7, wobei der Primärträger und/oder der Sekundärträger aus Glas oder Kunststoff besteht.

9. Verfahren nach einem der Ansprüche 1 bis 8, wobei die Oberfläche des Primärträgers oder des Sekundärträgers eine der folgenden Substanzen aufweist: Collagen, Heparansulfat, Fibronectin, Polylysin, Laminin, Vitronectin, Entactin.

10. System zur Verwendung in einem Verfahren nach einem der Ansprüche 1 bis 9, das umfasst:
(a) einen Primärträger mit abtrennbaren Trägerabschnitten für adhärente Zellen, wobei der Primärträger in einem Behältnis angeordnet ist, das eine Kultivierung von Zellen auf einer Oberfläche des Primärträgers erlaubt;
(b) einen Sekundärträger, der zur Aufnahme und Fixierung eines Trägerabschnitts geeignet ist, so dass ein Hinüberwachsen von Zellen von einer Oberfläche des Trägerabschnitts auf eine benachbarte Oberfläche des Sekundärträgers durch den Übergangsbereich zwischen Trägerabschnitt und Sekundärträger im wesentlichen nicht behindert wird.

## Claims

1. Process for treating cell cultures, **characterized by** the following steps:
(a) providing a primary carrier comprising on a surface thereof adherent cells, wherein said surface is suitable for adherence and growth of the cells;
(b) culturing of the cells on the carrier under predetermined conditions for forming a cell layer on the surface of the primary carrier;
(c) separating a carrier section carrying a cell layer from the carrier obtained in step (b);
(d) positioning the carrier section separated in step (c) in a spatial relationship to a surface of a secondary carrier such that the cells can grow from the carrier section onto the secondary carrier, the surface of the secondary carrier being suitable for adherence and growth of the cells.

2. Process according to claim 1, wherein the primary carrier can be divided into a plurality of carrier sections that can be positioned according to step (d).

3. Process according to claim 1 or 2, wherein a container is provided for the primary carrier and/or the secondary carrier, said container containing a nutrient medium or a buffer for said cells, and wherein one or more test substances can be added to the nutrient medium in the container of the secondary carrier.

4. Process according to any of claims 1 to 3, wherein growth of said cells from a carrier section onto the secondary carrier and/or propagation of the cells on the secondary carrier and/or a property of the cells propagating on a secondary carrier is determined.

5. Process according to any of claims 1 to 4, wherein, in step (c), two or more carrier sections of the primary carrier are separated that are positioned, in step (d), on different secondary carriers the surfaces of which are similar.

6. Process according to any of claims 1 to 5, wherein growth of said cells from a carrier section on the secondary carriers and/or propagation of said cells on the secondary carriers and/or a property of the cells propagating on a secondary carrier is monitored in the presence and in the absence of a test substance for determining an effect of the test substance on the cells.

7. Process according to any of claims 1 to 6, wherein steps (a) to (c) are carried out with two or more types of cells, each separately on a primary carrier, and wherein carrier sections having cell layers of different cell types are positioned on
(a) different positions of a secondary carrier or
(b) equivalent positions of different secondary carriers having similar surfaces.

8. Process according to any of claims 1 to 7, wherein the primary carrier and/or the secondary carrier is made of glass or plastic.

9. Process according to any of claims 1 to 8, wherein the surface of the primary carrier or of the secondary carrier comprises any of the following substances: collagen, heparan sulfate, fibronectin, polylysine, laminin, vitronectin, entactin.

10. System for use in a process according to any of claims 1 to 9, comprising:
(a) a primary carrier having detachable carrier sections for adherent cells, said primary carrier being located in a container allowing culturing of cells on a surface of the primary carrier;
(b) a secondary carrier adapted for receiving and fixing a carrier section such that growth of cells from a surface of the carrier section on an adjacent surface of the secondary carrier is essentially not disturbed by the transition region between carrier section and secondary carrier.

## Revendications

1. Procédé de traitement de cultures cellulaires, **caractérisé par** les étapes suivantes consistant à :
(a) préparer un support primaire comprenant des cellules qui adhèrent à une surface, ladite surface étant adaptée pour l'adhérence et la croissance des cellules ;
(b) mettre en culture les cellules sur le support dans des conditions prédéterminées afin de former une couche.de cellules à la surface du support primaire ;
(c) séparer une fraction de support du support obtenu à l'étape (b), ladite fraction portant une couche de cellules ;
(d) positionner la fraction de support séparée à l'étape (c), dans une disposition telle par rapport à une surface d'un support secondaire adaptée pour l'adhérence et la croissance des cellules, que les cellules peuvent croître sur le support secondaire depuis la fraction de support.

2. Procédé selon la revendication 1, dans lequel le support primaire peut être subdivisé en une pluralité de fractions de support qui peuvent être positionnées selon l'étape (d).

3. Procédé selon l'une des revendications 1 et 2, dans lequel un récipient est prévu pour le support primaire et/ou le support secondaire, ledit récipient contenant un milieu de culture ou un tampon pour les cellules et dans lequel une ou plusieurs substances de test peuvent être ajoutées au milieu de culture dans le récipient du support secondaire.

4. Procédé selon l'une des revendications 1 à 3, dans lequel on détermine la croissance des cellules sur le support secondaire depuis une fraction de support et/ou la propagation des cellules sur le support secondaire et/ou l'état des cellules qui se propagent sur le support secondaire.

5. Procédé selon l'une des revendications 1 à 4, dans lequel à l'étape (c), deux ou plus de deux fractions de support du support primaire sont séparées, lesdites fractions pouvant être positionnées à l'étape (d) sur différents supports secondaires dont les surfaces sont similaires.

6. Procédé selon l'une des revendications 1 à 5, dans lequel on suit la croissance des cellules sur le support secondaire depuis une fraction de support et/ou la propagation des cellules sur les supports secondaires et/ou l'état des cellules qui se propagent sur un support secondaire en présence et en l'absence d'une substance de test afin de déterminer un effet de la substance de test sur les cellules.

7. Procédé selon l'une des revendications 1 à 6, dans lequel les étapes (a) à (c) sont exécutées avec deux ou plus de deux types de cellules respectivement séparés sur un support primaire, et des fractions de support comprenant des couches de cellules de différents types sont positionnées dans
(a) différentes positions d'un support secondaire ou
(b) des positions équivalentes de différents supports secondaires ayant des surfaces similaires.

8. Procédé selon l'une des revendications 1 à 7, dans lequel le support primaire et/ou le support secondaire est (sont) constitué(s) de verre ou de matière plastique.

9. Procédé selon l'une des revendications 1 à 8, dans lequel la surface du support primaire ou du support secondaire comprend l'une des substances suivantes : collagène, sulfate d'héparane, fibronectine, polylysine, laminine, vitronectine, entactine.

10. Système pour une utilisation dans un procédé selon l'une des revendications 1 à 9, lequel système comprend :
(a) un support primaire comprenant des fractions de support séparables pour des cellules adhérentes, dans lequel le support primaire est agencé dans un récipient qui permet une mise en culture des cellules sur une surface du support primaire ;
(b) un support secondaire qui est adapté pour recevoir et fixer une fraction de support, de telle sorte qu'une croissance de cellules depuis une surface de la fraction de support sur une surface voisine du support secondaire n'est pratiquement pas gênée par la zone de transition entre la fraction de support et le support secondaire.
